# EUROPÄISCHE PATENTSCHRIFT

(11) **EP 0 649 012 B1**
(45) Veröffentlichungstag und Bekanntmachung des Hinweises auf die Patenterteilung: **19.01.2000**
(21) Anmeldenummer: 94115807.3
(22) Anmeldetag: 07.10.1994
(51) Int. Cl.: G01N 11/16, G01N 33/49, G01N 33/543

(54) **Sensor zum Messen von Viskositäts- und/oder Dichteänderungen**
Sensor for measuring changes of viscosity and/or density
Capteur pour mesurer des variations de viscosité et/ou de densité

(30) Priorität: 13.10.1993 DE 4334834
(43) Veröffentlichungstag der Anmeldung: 19.04.1995
(73) Patentinhaber: Dade Behring Marburg GmbH, 35041 Marburg (DE)
(72) Erfinder: Grzegorzewski, Andrzej, Dr., D-28832 Achim-Uphusen (DE)

(56) Entgegenhaltungen:
- EP-A- 0 304 283
- EP-B- 0 177 858
- WO-A-91/01381
- DE-A- 3 920 052
- US-A- 4 735 906
- US-A- 5 201 215
- ANALYTICAL CHEMISTRY, Bd.61, Nr.11, 1. Juni 1989, WASHINGTON, DC, US Seiten 1227 - 1230, XP33995 K. A. DAVIS ET AL.

## Beschreibung

Die Erfindung betrifft einen Sensor zum Messen von Viskositäts- und/oder Dichteänderungen eines zu untersuchenden Fluids, insbesondere zur Messung der Gerinnung von Blut, mit einem eine Meßkammer umschließenden Gehäuse, in der ein Piezoelement, insbesondere ein als Scherschwinger ausgebildeter Quarzkristall, als Schwingkreis angeordnet ist, von dem eine Meßoberfläche mit dem zu untersuchenden Fluid und einer Reaktionskomponente benetzt wird und Änderungen der Schwingkreisparameter durch eine entsprechende elektronische Auswerteschaltung ausgewertet werden.

Aus der EP-B-177 858 ist eine Anordnung mit einem Sensor bekannt, mit der die Gerinnungszeit von Blut gemessen werden soll. In einer mit einem Deckel verschließbaren Meßkammer ist ein Quarzkristall angeordnet, der als Resonanzkreis an einen Oszillator mit fester Frequenz angeschlossen ist. Das zu untersuchende Blut wird vorher mit einer Gerinnungskomponente vermischt und auf die Meßoberfläche des Quarzkristalls appliziert, und es wird anschließend die Gerinnungszeit dadurch gemessen, daß eine Amplitudenabnahme am Quarzkristall aufgrund der Bedämpfung oder Verstimmung des Resonanzkreises durch das gerinnende Blut ausgewertet wird. Die bis zur Gerinnung verstreichende Zeit wird durch eine elektronische Stoppuhr gemessen. Dieser bekannte Sensor hat den Nachteil, daß nach einem erfolgten Meßvorgang seine Meßkammer bzw. die Meßoberfläche des Quarzkristalls nur sehr schwer zu reinigen ist, und zum anderen die vom Zeitpunkt des Vermischens des Blutes mit einer Gerinnungskomponente verstreichende Zeit bis zum Aufbringen auf die Meßoberfläche sehr sorgfältig berücksichtigt und kontrolliert werden muß, um Fehlmessungen zu vermeiden. Außerdem ist das vorher notwendige Mischen der zu untersuchenden Flüssigkeit verhältnismäßig umständlich. Ähnliche Vorrichtungen zeigen auch die japanischen Patentpublikationen JP-A-62/153761 und JP-A-4/32767.

Darüber hinaus sind Sensoren zum Erkennen von Antigen-Antikörper-Reaktionen bekannt, die mit Piezoelementen, z.B. einem Quarzkristall, arbeiten. Bei solchen Sensoren ist auf der Meßoberfläche des Piezoelementes ein Antigen oder Antikörper aufgebracht, und das Piezoelement wird anschließend in das zu untersuchende Fluid zur Messung der anderen Komponente eingetaucht, um die dabei auftretende Verstimmung des Piezoelementes als Schwingkreis aufgrund von Masseanlagerungen auf der Sensoroberfläche zu messen und auszuwerten. Solche Sensoren sind z.B. in den US-Patentschriften 4,236,893 und 4,735,906 beschrieben.

Doch bei diesen bekannten Sensoren mit einem Antigen oder Antikörper auf der Meßoberfläche tritt das Problem auf, daß die selektiv absorbierende Schicht vor jeder neuen Benutzung gereinigt werden muß, was sehr aufwendig ist und die Schicht allmählich zerstört. Eine Übertragung dieses Prinzips auf einen Blutgerinnungssensor, bei dem also die Reaktionskomponente sich bereits auf der Meßoberfläche befände, hätte den Nachteil, daß die Meßoberfläche des Piezoelementes bzw. Quarzes bereits mit der Reaktionskomponente belastet ist, so daß eine Messung der Schwingfrequenz des Piezoelementes im unbelasteten Zustand nicht mehr möglich ist.

Der vorliegenden Erfindung liegt die Aufgabe zugrunde, einen Sensor zum Messen von Viskositäts- und/oder Dichteänderungen eines zu untersuchenden Fluids vorzuschlagen, der einfach und damit kostensparend aufgebaut ist, kein vorheriges Zugeben einer Reaktionskomponente zu den zu untersuchenden Fluid erfordert sowie keinerlei Reinigungsprobleme nach Gebrauch aufwirft.

Diese Aufgabe wird gemäß der vorliegenden Erfindung dadurch gelöst, daß der Sensor als Einwegteil ausgebildet ist, bei dem innerhalb der Meßkammer die Reaktionskomponente in der Nähe der Meßoberfläche des Piezoelementes, aber diese noch nicht berührend, derart untergebracht ist, daß beim Einbringen des zu untersuchenden Fluids über einen Zugang dieses mit der Reaktionskomponente und mit der Meßoberfläche des Piezoelementes in Berührung kommt.

Zur Ausführung dieser erfindungsgemäßen Lösung kommen mehrere, vorteilhafte Ausführungsformen in Betracht. In einer ersten Lösungsgruppe besteht der Sensor vorzugsweise sandwichartig aus mehreren Schichten, die zweckmäßigerweise zusammengeklebt sind. Das Gehäuse besteht vorzugsweise aus fünf Schichten, nämlich einer ersten, einen geschlossenen Boden bildenden Schicht, einer zweiten, einen Freiraum unter dem Piezoelement lassenden Schicht, einer dritten, das Piezoelement halternden Schicht, einer vierten, die Meßkammer über dem Piezoelement bildenden Schicht, sowie einer fünften, die Meßkammer über dem Piezoelement als Deckel abschließenden Schicht mit einem Zugang zum Einfüllen des zu untersuchenden Fluids in die Meßkammer.

Eine weitere Gruppe von vorteilhaften Ausführungsformen z.B. dadurch gekennzeichnet, daß das Gehäuse aus zwei zusammengesetzten, insbesondere miteinander verklebten Gehäuseschalen besteht, in die das Piezoelement eingesetzt ist. Vorzugsweise ist die Ausbildung derart, daß in die eine Gehäuseschale, die den Zugang aufweist, das Piezoelement in eine entsprechende Aussparung eingesetzt, insbesondere eingeklebt ist, und daß die andere, den Boden bildende Gehäuseschale einen Vorsprungsabschnitt hat, der auf dem Rand des Piezoelementes aufliegt. Eine Variante kann zweckmäßigerweise darin bestehen, daß das Piezoelement auf einem Trägerteil angeordnet ist, das zwischen den beiden Gehäuseschalen eingefaßt ist.

Es ist jedoch auch möglich, das Gehäuse einstückig mit einer von außen zugänglichen Kammer auszubilden, wobei in diese Kammer ein die Reaktionskomponente tragender Träger und das Piezoelement über einen Einführungskanal eingesetzt und vorzugsweise verklebt sind. Die Gehäuseschalen (der vorherigen Ausführungsformen) bzw. das Gehäuse der letzten Ausführungsform sind vorzugsweise aus Kunststoff gespritzt.

Um das zu untersuchende Fluid in die Meßkammer einzubringen, gibt es mehrere Möglichkeiten. Eine Lösung besteht darin, daß der Zugang zum Einbringen des zu untersuchenden Fluids aus einer mit der Meßkammer in Verbindung stehenden Bohrung besteht. Eine andere Lösung besteht darin, daß der Zugang zum Einbringen des zu untersuchenden Fluids aus einer die Meßkammer abdeckenden, für das Fluid durchlässigen Membrane besteht.

Die Unterbringung der Reaktionskomponente für die Reaktion mit dem zu untersuchenden Fluid kann z.B. derart erfolgen, daß die Reaktionskomponente auf die Wandungen der Meßkammer aufgebracht ist. Eine andere, vorteilhafte Lösung besteht darin, daß die Reaktionskomponente auf einem Träger enthalten ist, der in die Meßkammer derart eingesetzt ist, daß er die Meßoberfläche nicht berührt. Der Träger kann vorzugsweise ein saugfähiges Kissen aus z.B. Watte oder Filterpapier sein. Es ist jedoch auch möglich, als Träger ein Gitter, z.B. aus Kunststoff oder Metall zu verwenden. Statt einer Reaktionskomponente können auch mehrere verschiedene Komponenten in der Meßkammer untergebracht sein.

Die Halterung und der Anschluß des Piezoelementes kann z.B. dadurch erfolgen, daß dieses in einer Trägerplatte gehaltert und über auf die Trägerplatte aufgedruckte Leiterbahnen mit aus dem Gehäuse herausgeführten Anschlußflächen verbunden ist.

Für den Fall, daß der Sensor als Blutgerinnungssensor ausgebildet ist, ist als Reaktionskomponente CaCl₂ oder eine andere Substanz zum Beeinflußen des Gerinnungsablaufs vorgesehen. Um zu besonders stabilen Messungen zu kommen, ist der Sensor vorzugsweise mit einer Einrichtung zum Temperieren versehen, um die Temperatur auf einem konstanten Wert zu halten.

Eine bevorzugte Meßanordnung mit einem Sensor gemäß der Erfindung ist dadurch gekennzeichnet, daß eine Oszillatorschaltung sowie eine Mikroprozessorschaltung als elektronische Auswerteschaltung vorgesehen sind und daß das Piezoelement als frequenzbestimmendes Element in die Oszillatorschaltung eingefügt ist und die Frequenzänderungen digital durch die Mikroprozessorschaltung gemessen und ausgewertet werden. Die Mikroprozessorschaltung kann in einem solchen Fall die Zeitkomponente bei der Messung berücksichtigen und durch entsprechende Programmierung eine qualitative und/oder quantitative Beurteilung vornehmen. Mit einer solchen Meßanordnung mit Mikroprozessorschaltung ist es möglich, die Temperaturabängigkeit des Piezoelementes zum Messen der Temperatur im Sensor auszunutzen, um davon abhängig die Auswertung zu steuern oder ein Temperieren des Sensors auf eine konstante Temperatur durchzuführen.

Es wird noch hinzugefügt, daß der Sensor zum Messen von Viskositäts- und/oder Dichteänderungen eines zu untersuchenden Fluids für alle fließfähigen Medien geeignet und angepaßt werden kann, also für Flüssigkeiten, pastöse Massen sowie für Gase. Neben der Messung der Gerinnung von Blut und von Antigen-Antikörper-Reaktionen sind auch Messungen von Viskositäts- und/oder Dichteänderungen auf anderen Gebieten möglich.

Die Erfindung wird nachfolgend anhand von Ausführungsbeispielen unter Bezug auf die beigefügten Zeichnungen näher erläutert.

Es zeigen:
- Figur 1: einen Querschnitt durch einen Sensor gemäß der Erfindung in Sandwichbauweise;
- Figur 2: eine Draufsicht auf eine das Piezoelement tragende Schicht des Sensors nach Figur 1;
- Figur 3: einen Querschnitt durch einen Sensor mit zwei Gehäusehälften;
- Figur 4: einen Querschnitt durch eine Variante der Ausführungsform nach Figur 3;
- Figur 5: einen Querschnitt durch einen Sensor mit einstückigem Gehäuse; und
- Figur 6: ein Blockschaltbild einer Meßanordnung mit dem erfindungsgemäßen Sensor.

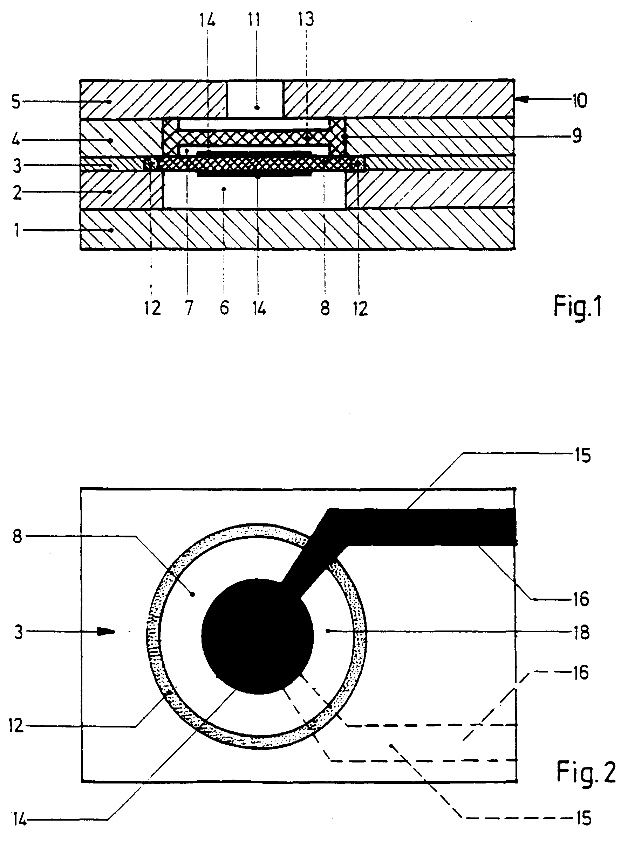

Der in Figur 1 gezeigte Sensor in Sandwichbauweise enthält fünf Schichten. Eine erste Schicht 1 bildet den Boden des Sensors 10, eine zweite Schicht 2 schafft durch eine entsprechende Aussparung einen Freiraum 6 unter einem Piezoelement 8, eine dritte Schicht 3 dient als Träger für das Piezoelement 8, eine vierte Schicht bildet durch eine entsprechende Aussparung 9 eine Meßkammer 7 über dem Piezoelement 8 und schließlich bildet eine fünfte Schicht 5 einen Abschluß für die Meßkammer 7 über dem Piezoelement 8, wobei ein Zugang 11 in Form einer Bohrung das Einfüllen des zu untersuchenden Fluids in die Meßkammer 7 zuläßt. Es ist auch möglich, statt der abdeckenden Schicht 5 mit Bohrung 11 eine für das zu untersuchende Fluid durchlässige Membran vorzusehen.

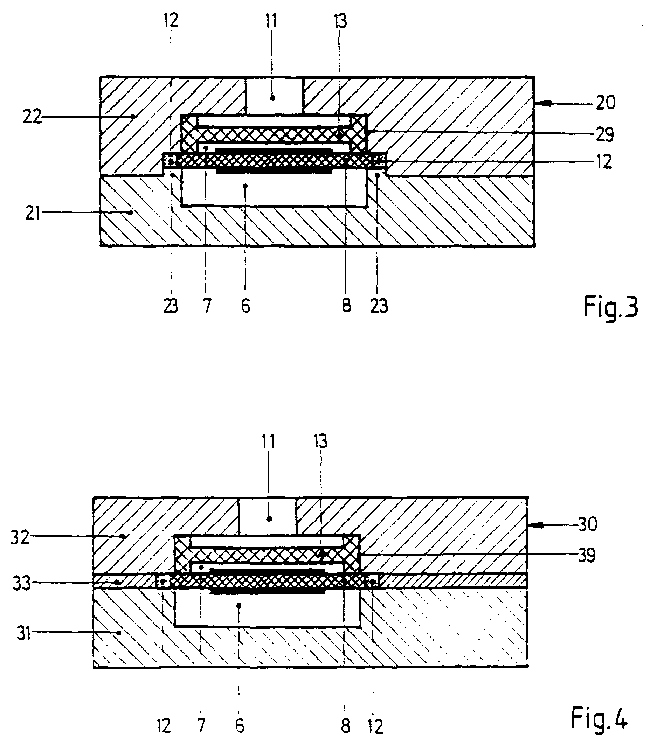

Wie auch aus Figur 2 hervorgeht, ist das Piezoelement 8 auf einer die dritte Schicht 3 bildenden Trägerplatte angeordnet, und zwar ist das Piezoelement 8 mittels eines Klebers 12 in eine entsprechende Aussparung in der dritten Schicht 3 eingeklebt. Beide Flächen des Piezoelementes 8 sind mit Elektroden 14 versehen, die über auf die dritte Schicht 3 aufgebrachte Leiterbahnen 15 mit Anschlußflächen 16 verbunden sind. Die (in Figur 1 gesehen) obere Fläche, in Figur 2 mit dem Bezugszeichen 18 versehen, bildet die Meßoberfläche des Piezoelementes 8.

In die Meßkammer 7 (siehe Figur 1) des Sensors 10, also in die Aussparung 9, ist ein Träger 13 derart eingesetzt, daß er die Meßoberfläche 18 des Piezoelementes 8 nicht berührt. Der Träger 13 dient zur Aufnahme einer Reaktionskomponente, mit der das zu untersuchende Fluid reagieren soll. Beim Einfüllen des zu untersuchenden Fluids über den Zugang bzw. die Bohrung 11 reagiert das Fluid mit der im Träger 13 befindlichen Reaktionskomponente und erreicht die Meßoberfläche 18 des Piezoelementes 8. Diese bedeutet also, daß der Meßbeginn mit dem Einfüllen des zu untersuchenden Fluids exakt festgelegt wird, und es nicht wie bisher auf die Geschicklichkeit der Bedienungsperson ankommt, wie schnell sie das zu untersuchende Fluid mit einer Reaktionskomponente vermischt und dann in einen Sensor oder dergleichen einfüllt.

Auch wenn in den vorliegenden Ausführungsbeispielen die Reaktionskomponte in einem Träger 13 angeordnet ist, der in die Meßkammer 7 eingesetzt ist, so ist es auch möglich, die Reaktionskomponente in geeigneter Weise auf den Oberflächen der Wandungen der Meßkammer 7 aufzubringen, wobei jedoch darauf geachtet werden muß, daß die Meßoberfläche 18 des Piezoelementes 8 noch nicht benetzt wird. Es ist auch möglich, anstelle von nur einer Reaktionskomponente mehrere, z.B. zwei, Reaktionskomponenten in der Meßkammer 7 anzuordnen, z.B. mittels mehrerer Träger 13 oder an getrennten Wandungsabschnitten der Meßkammer 7.

Der in den Figuren 1 und 2 dargestellte Sensor 10 in Sandwichform kann auf geeignete Weise, z.B. durch Verkleben der einzeinen Schichten 1-5 hergestellt werden. Die einzelnen Schichten 1 bis 5 bestehen vorzugsweise aus Kunststoffolien; es ist jedoch auch die Verwendung von Papier für einige der Schichten möglich. Außerdem ist es möglich, einige der Schichten 1-5 zusammenzufassen, z.B. die Schichten 1 und 2 sowie 4 und 5, wobei die jeweiligen Aussparungen für den Freiraum 6 bzw. die Meßkammer 7 durch Prägen hergestellt werden.

Bei der Ausführungsform eines Sensors 20 nach Figur 3 besteht ein Gehäuse aus zwei Gehäuseschalen 21 und 22, die zusammengeklebt sind. Die Gehäuseschale 21 bildet den Boden sowie den Freiraum 6 unter dem Piezoelement 8, während die Gehäuseschale 22 das Oberteil mit einer Aussparung 29 für die Meßkammer 7 und einer Bohrung als Zugang 11 bildet. Die Aussparung 29 in der oberen Gehäuseschale 22 ist so geformt, daß sie zum einen den Träger 13 für die Reaktionskomponente als auch das Piezoelement 8 aufnimmt, daß mittels eines Klebers 12 in die obere Gehäuseschale 22 eingeklebt ist. Beim Aufsetzen der unteren Gehäuseschale 21 drücken Vorsprungsabschnitte 23 auf den äußeren Bereich des Piezoelementes 8 und spannen dieses zusammen mit den entsprechenden Bereichen der oberen Gehäuseschale 22 ein. Es ist also zu sehen, daß der Sensor 20 nach Figur 3 auf einfache Weise montiert werden kann.

Eine weitere Ausführungsform zeigt Figur 4. Der Sensor 30 besteht ebenfalls aus einer unteren Gehäuseschale 31 und einer oberen Gehäuseschale 32, die jedoch zwischen sich ein Trägerteil 33 für das Piezoelement 8 zwischen sich einschließen und miteinander verklebt sind. Das Piezoelement 8 ist nicht wie bei der Ausführungsform nach Figur 3 in die obere Gehäuseschale eingeklebt, sondern es wird in einer entsprechenden Aussparung im Trägerteil 13 durch einen Kleber 12 gehaltert. Eine Aussparung 39 in der oberen Gehäuseschale 32 haltert nur den Träger 13 für die Reaktionskomponente und bildet im übrigen die Meßkammer 7.

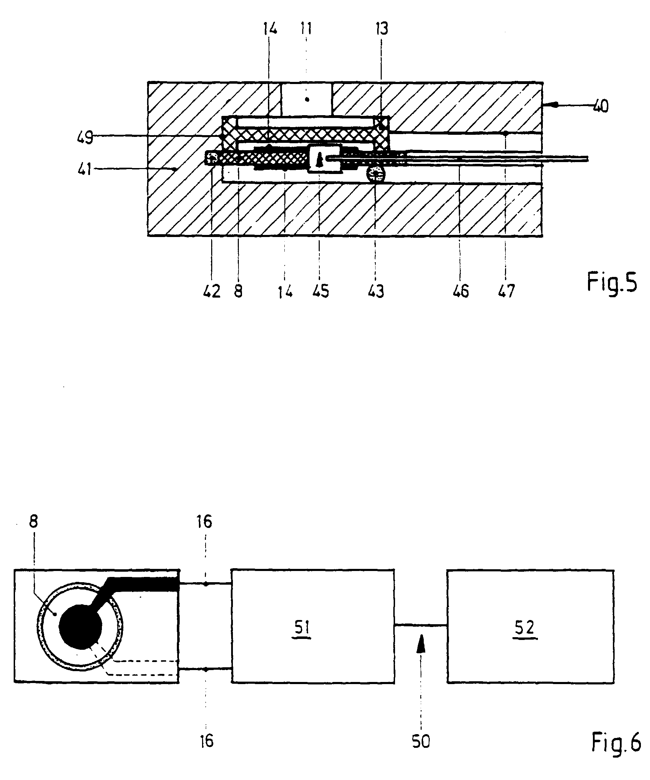

Figur 5 zeigt nun eine weitere Form eines Sensors 40. Der Sensor 40 weist ein einstückiges Gehäuse 41 auf, das mit einer inneren Kammer 49 und einem Einführungskanal 47 versehen ist. Die Kammer 49 ist derart geformt, daß sowohl der Träger 13 für die Reaktionskomponente als auch eine Kontaktklemme 45 mit dem Piezoelement 8 aufgenommen und sicher gehaltert werden kann; außerdem besteht über den Zugang 11 eine entsprechende Öffnung nach außen zum Einfüllen des zu untersuchenden Fluids.

Das Piezoelement 8 mit der Kontaktklemme 45 ist durch Kleber 42, 43 nach dem Einsetzen durch den Einführungskanal 47 im Innern des Gehäuses 41 befestigt. Die Elektroden 14 des Piezoelementes 8 sind über Anschlußdrähte 46 herausgeführt.

Die Gehäuseschalen 21, 22 bzw. 31, 32 sowie das einstückige Gehäuse 41 der drei Ausführungsformen nach den Figuren 3, 4 und 5 werden zweckmäßigerweise im Spritzguß aus einem geeigneten Kunststoff hergestellt. Die Herstellung ist sehr einfach und kostensparend, so daß nicht nur der Sensor 10 nach der ersten Ausführungsform, sondern auch die Ausführungsformen der Sensoren 20, 30 und 40 als Einweg-Sensoren hergestellt und eingesetzt werden können. Die Einwegform hat vor allem den Vorteil, daß die Sensoren 10, 20, 30 und 40 fertig montiert und mit der Reaktionskomponente versehen ausgeliefert werden können; eine Wiederverwendung wäre bei einem solchen Aufbau nicht sinnvoll.

Die in Figur 6 gezeigte Meßanordnung enthält eine Auswerteschaltung 50, die eine Oszillatorschaltung 51, eine Mikroprozessorschaltung 52 sowie den Sensor aufweist, dessen Piezoelement 8 über die Anschlußflächen 16 mit der Oszillator-Biosschaltung 51 verbunden ist. Die Oszillatorschaltung 51 benutzt das Piezoelement 8, das zweckmäßigerweise ein Quarzkristall ist, als frequenzbestimmendes Element, während die Mikroprozessorschaltung 52 eine entsprechende Frequenzmeßanordnung zum Messen der Schwingungsfrequenz des Oszillators 51 aufweist.

Der Mikroprozessor 52 kann außerdem so programmiert und ausgebildet sein, daß er die Frequenzänderungen oder sonstige Parameter des Piezoelementes 8 bzw. der Oszillatorschaltung 51 auswertet und dabei die Zeitkomponente der entsprechenden Änderungen berücksichtigt. Durch entsprechende Programmierung kann aus diesen Meßwerten eine geeignete Auswertung erfolgen und auf einem Display oder einer anderen Ausgabeeinheit (nicht dargestellt) ausgegeben werden.

## Patentansprüche

1. Sensor zum Messen von Viskositäts- und/oder Dichteänderungen eines zu untersuchenden Fluids, insbesondere zur Messung der Gerinnung von Blut, mit einem eine Meßkammer umschließenden Gehäuse, in der ein Piezoelement, insbesondere ein als Scherschwinger ausgebildeter Quarzkristall, als Schwingkreis angeordnet ist, von dem eine Meßoberfläche mit dem zu untersuchenden Fluid und einer Reaktionskomponente benetzt wird und Änderungen der Schwingkreisparameter durch eine entsprechende elektronische Auswerteschaltung ausgewertet werden,
dadurch gekennzeichnet,
daß der Sensor (10, 20, 30, 40) als Einwegteil ausgebildet ist, bei dem innerhalb der Meßkammer (7) die Reaktionskomponente in der Nähe der Meßoberfläche (18) des Piezoelementes (8), aber diese noch nicht berührend, derart untergebracht ist,
daß beim Einbringen des zu untersuchenden Fluids über einen Zugang (11) dieses mit der Reaktionskomponente und mit der Meßoberfläche (18) des Piezoelementes (8) in Berührung kommt.

2. Sensor nach Anspruch 1,
dadurch gekennzeichnet, daß er sandwichartig aus mehreren Schichten (1, 2, 3, 4, 5) zusammengesetzt, insbesondere zusammengeklebt, ist.

3. Sensor nach Anspruch 2,
dadurch gekennzeichnet, daß des Gehäuse aus fünf Schichten (1-5) besteht, nämlich
- einer ersten, einen geschlossenen Boden bildenden Schicht (1),
- einer zweiten, einen Freiraum (6) unter dem Piezoelement (8) lassenden Schicht (2),
- einer dritten, des Piezoelement (8) halternden Schicht (3),
- einer vierten, die Meßkammer (7) über dem Piezoelement (8) bildenden Schicht (4),
- einer fünften, die Meßkammer (7) über dem Piezoelement (8) als Deckel abschileßenden Schicht (5) mit einem Zugang (11) zum Einfüllen des zu untersuchenden Fluids in die Meßkammer (7).

4. Sensor nach Anspruch 1,
dadurch gekennzeichnet, daß des Gehäuse aus zwei zusammengesetzten, insbesondere miteinander verklebten, Gehäuseschalen (21, 22) besteht, in die des Piezoelement (8) eingesetzt ist.

5. Sensor nach Anspruch 4,
dadurch gekennzeichnet, daß in die eine Gehäuseschale (22), die den Zugang (11) aufweist, des Piezoelement (8) in eine entsprechende Aussparung (29) eingesetzt, insbesondere eingeklebt ist, und daß die andere, den Boden bildende Gehäuseschale (21) einen Vorsprungsabschnitt (23) hat, der auf dem Rand des Piezoelementes (8) aufliegt.

6. Sensor nach Anspruch 4,
dadurch gekennzeichnet, daß das Piezoelement (8) auf einem Trägerteil (33) angeordnet ist, des zwischen den beiden Gehäuseschalen (31, 32) eingefaßt ist.

7. Sensor nach Anspruch 1,
dadurch gekennzeichnet, daß des Gehäuse (41) einstückig mit einer von außen zugänglichen Kammer (49) ausgebildet ist und daß in diese Kammer (49) ein die Reaktionskomponente tragender Träger (13) und des Piezoelement (8) über einen Einführungskanal (47) eingesetzt und vorzugsweise verklebt sind.

8. Sensor nach einem der Ansprüche 5 bis 7,
dadurch gekennzeichnet, daß die Gehäuseschalen (21, 22; 31, 32) bzw. das Gehäuse (41) aus Kunststoff gespritzt sind.

9. Sensor nach einem der Ansprüche 1 bis 8,
dadurch gekennzeichnet, daß der Zugang zum Einbringen des zu untersuchenden Fluids aus einer mit der Meßkammer (7) in Verbindung stehenden Bohrung (11) besteht.

10. Sensor nach einem der Ansprüche 1 bis 8,
dadurch gekennzeichnet, daß der Zugang zum Einbringen des zu untersuchenden Fluids aus einer die Meßkammer (7) abdeckenden, für das Fluid durchlässigen Membrane besteht.

11. Sensor nach einem der Ansprüche 1 bis 6,
dadurch gekennzeichnet, daß die Reaktionskomponente auf die Wandungen der Meßkammer (7) aufgebracht ist.

12. Sensor nach einem der Ansprüche 1 bis 10,
dadurch gekennzeichnet, daß die Reaktionskomponente auf einem Träger (13) enthalten ist, der in die Meßkammer (7) derart eingesetzt ist, daß er die Meßoberfläche (18) nicht berührt.

13. Sensor nach Anspruch 12,
dadurch gekennzeichnet, daß als Träger (13) ein saugfähiges Kissen aus z.B. Watte oder Filterpapier verwendet ist.

14. Sensor nach Anspruch 12,
dadurch gekennzeichnet, daß als Träger (13) ein Gitter, z.B. aus Kunststoff oder Metall verwendet ist.

15. Sensor nach einem der vorstehenden Ansprüche,
dadurch gekennzeichnet, daß das Piezoelement (8) in einer Trägerplatte (3; 33) gehaltert und über auf die Trägerplatte aufgedruckte Leiterbahnen (15) mit aus dem Gehäuse herausgeführten Anschlußflächen (16) verbunden ist.

16. Sensor nach einem der Ansprüche 1 bis 15, der als Blutgerinnungssensor ausgebildet ist,
dadurch gekennzeichnet, daß als Reaktionskomponente CaCl₂ oder andere Substanzen zum Beeinflussen des Gerinnungsablaufs vorgesehen sind.

17. Sensor nach einem der vorstehenden Ansprüche,
dadurch gekennzeichnet, daß er mit einer Einrichtung zum Temperieren versehen ist, um die Temperatur auf einem konstanten Wert zu halten.

18. Meßanordnung mit einem Sensor nach einem der vorstehenden Ansprüche,
dadurch gekennzeichnet, daß sie eine Oszillatorschaltung (51) und eine Mikroprozessorschaltung (52) als elektronische Auswerteschaltung (50) enthält, daß das Piezoelement (8) als frequenzbestimmendes Element in die Oszillatorschaltung (51) eingefügt ist und daß Frequenzänderungen digital durch die Mikroprozessorschaltung (52) gemessen und ausgewertet werden.

19. Meßanordnung nach Anspruch 18,
dadurch gekennzeichnet, daß die Temperaturabhängigkeit des Piezoelementes (8) zum Messen der Temperatur ausgenutzt wird.

## Claims

1. A sensor for measuring changes in viscosity and/or density in a test fluid, in particular for measuring the coagulation of blood, having a housing which surrounds a measuring chamber in which a piezoelectric element, in particular a quartz crystal constructed as a shear-mode transducer, is arranged, as an oscillating circuit, a measuring surface of which is wetted with the test fluid and a reaction component, and changes in the oscillating circuit parameters are evaluated by a corresponding electronic evaluation circuit, wherein the sensor (10, 20, 30, 40) is constructed as a disposable part in which the reaction component is accommodated inside the measuring chamber (7) in the vicinity of the measuring surface (18) of the piezoelectric element (8), but not yet in contact with said surface, in such a way that upon being introduced via an access (11) the test fluid comes into contact with the reaction component and with the measuring surface (18) of the piezoelectric element (8).

2. The sensor as claimed in claim 1, wherein it is assembled, in particular glued together, like a sandwich from a plurality of layers (1, 2, 3, 4, 5).

3. The sensor as claimed in claim 2, wherein the housing comprises five layers (1-5), specifically
- a first layer (1) forming a closed bottom,
- a second layer (2), which leaves a free space under the piezoelectric element (8),
- a third layer (3), which holds the piezoelectric element (8),
- a fourth layer (4), which forms the measuring chamber (7) above the piezoelectric element (8), and
- a fifth layer (5), which, as a lid, exposes the measuring chamber (7) above the piezoelectric element (8), and has an access (11) for filling the test fluid into the measuring chamber (7).

4. The sensor as claimed in claim 1, wherein the housing comprises two assembled, in particular glued together, housing shells (21, 22) into which the piezoelectric element (8) is inserted.

5. The sensor as claimed in claim 4, wherein the piezoelectric element (8) is inserted, in particular glued, into one housing shell (22) which has the access (11), into a corresponding recess (29), and in that the other housing shell (21), which forms the bottom, has a projecting section (23) which rests on the edge of the piezoelectric element (8).

6. The sensor as claimed in claim 4, wherein the piezoelectric element (8) is arranged on a substrate part (33) which is mounted between the two housing shells (31, 32).

7. The sensor as claimed in claim 1, wherein the housing (41) is constructed in one piece with a chamber (49) accessible from outside, and in that a substrate (13), carrying the reaction component, and the piezoelectric element (8) are inserted into this chamber (49) via an introduction conduit (47), and preferably glued.

8. The sensor as claimed in one of claims 5 to 7, wherein the housing shells (21, 22; 31, 32) and/or the housing (41) are made from injection-molded plastic.

9. The sensor as claimed in one of claims 1 to 8, wherein the access for introducing the test fluid comprises a bore (11) connected to the measuring chamber (7).

10. The sensor as claimed in one of claims 1 to 8, wherein the access for introducing the test fluid comprises a membrane covering the measuring chamber (7) and permeable to the fluid.

11. The sensor as claimed in one of claims 1 to 6, wherein the reaction component is applied to the walls of the measuring chamber (7).

12. The sensor as claimed in one of claims 1 to 10, wherein the reaction component is contained on a substrate (13) which is inserted into the measuring chamber (7) in such a way that it does not make contact with the measuring surface (18).

13. The sensor as claimed in claim 12, wherein use is made as substrate (13) of an absorbent pad made from, for example, cotton wool or filter paper.

14. The sensor as claimed in claim 12, wherein use is made as substrate (13) of a grid made from, for example, plastic or metal.

15. The sensor as claimed in one of the preceding claims, wherein the piezoelectric element (8) is held in a substrate board (3; 33) and is connected to terminal faces (16), led out of the housing, via conductor tracks (15) printed onto the substrate board.

16. The sensor as claimed in one of claims 1 to 15, which is constructed as a blood coagulation sensor, wherein CaCl₂ or other substances for influencing the course of coagulation are provided as reaction component.

17. The sensor as claimed in one of the preceding claims, wherein it is provided with a device for temperature control in order to keep the temperature at a constant value.

18. A measuring arrangement having a sensor as claimed in one of the preceding claims, wherein it includes an oscillator circuit (51) and a microprocessor circuit (52) as electronic evaluation circuit (50), wherein the piezoelectric element (8) is inserted as frequency-determining element into the oscillator circuit (51) and wherein the frequency changes are measured and evaluated digitally by the microprocessor circuit (52).

19. The measuring arrangement as claimed in claim 18, wherein the temperature dependence of the piezoelectric element (8) is utilized to measure the temperature.

## Revendications

1. Capteur pour mesurer des variations de viscosité et/ou de densité d'un fluide à étudier, en particulier pour la mesure de la coagulation du sang, comprenant un boîtier qui renferme une chambre de mesure, dans laquelle un piézo-élément, en particulier un cristal de quartz constitué par un oscillateur de cisaillement, est disposé en tant que circuit oscillant, dont une surface de mesure est mouillée par le fluide à étudier et par un composant de réaction, et les variations des paramètres du circuit oscillant sont analysées par un circuit d'analyse électronique correspondant,
caractérisé en ce que le capteur (10, 20, 30, 40) est constitué par un élément à usage unique, dans lequel, à l'intérieur de la chambre de mesure (7), le composant de réaction est logé dans le voisinage de la surface de mesure (18) du piézo-élément (8) mais sans la toucher, de telle manière que, lorsqu'on introduit le fluide à mesurer par une voie d'accés (11), ce fluide entre en contact avec le composant de réaction et avec la surface de mesure (18) du piézo-élément (8).

2. Capteur selon la revendication 1,
caractérisé en ce qu'il est composé, à la façon d'un stratifié, de plusieurs couches (1, 2, 3, 4, 5) en particulier par collage.

3. Capteur selon la revendication 2,
caractérisé en ce que le boîtier est composé de cinq couches (1-5), à savoir
une première couche (1) qui forme un fond fermé,
une deuxième couche (2) qui ménage un espace libre (6) sous le piézo-élément (8),
une troisième couche (3) qui tient le piézo-élément (8),
une quatrième couche (4) qui forme la chambre de mesure (7) au-dessus du piézo-élément (8),
une cinquième couche (5) qui ferme la chambre de mesure (7) située au-dessus du piézo-élément (8) pour former un couvercle, et qui présente un accès (11) pour l'introduction du fluide à étudier dans la chambre de mesure (7).

4. Capteur selon la revendication 1,
caractérisé en ce que le boîtier est constitué par deux coques de boîtier (21, 22) assemblées, en particulier collées l'une à l'autre, dans lesquelles le piézo-élément (8) est inséré.

5. Capteur selon la revendication 4,
caractérisé en ce que le piézo-élément (8) est introduit, en particulier collé, dans l'une (22) des coques du boîtier, qui présente l'accès (11), et logé dans un évidement (29) approprié, et en ce que l'autre coque (21) du boîtier, qui forme le fond, possède une section en saillie (23) qui repose sur le bord du piézo-élément (8).

6. Capteur selon la revendication 4,
caractérisé en ce que le piézo-élément (8) est disposé sur un élément support (33) qui est serré entre les deux coques (31, 32) du boîtier.

7. Capteur selon la revendication 1,
caractérisé en ce que le boîtier (41) est réalisé en une seule pièce avec une chambre (49) accessible de l'extérieur, et en ce qu'un support (13) portant le composant de réaction et le piézo-élément (8) sont introduits en passant par un canal d'entrée (47), et de préférence collés dans cette chambre (49).

8. Capteur selon l'une des revendications 5 à 7,
caractérisé en ce que les coques (21, 22 ; 31, 32) du boîtier, ou le boîtier (41), sont moulés par injection en matière plastique.

9. Capteur selon l'une des revendications 1 à 8,
caractérisé en ce que l'accès pour l'introduction du fluide à étudier est constitué par un perçage (11) qui est en communication avec la chambre de mesure (7).

10. Capteur selon l'une des revendications 1 à 8,
caractérisé en ce que l'accès pour l'introduction du fluide à étudier est constitué par une membrane perméable à ce fluide et qui recouvre la chambre de mesure (7).

11. Capteur selon l'une des revendications 1 à 8,
caractérisé en ce que le composant de réaction est déposé sur les parois de la chambre de mesure (7).

12. Capteur selon l'une des revendications 1 à 10, caractérisé en ce que le composant de réaction est contenu sur un support (13) qui est inséré dans la chambre de mesure (7) de maniéré qu'il ne touche pas la surface de mesure (18).

13. Capteur selon la revendication 12,
caractérisé en ce qu'on utilise comme support (13), un coussin absorbant par exemple en ouate ou en papier filtre.

14. Capteur selon la revendication 12,
caractérisé en ce qu'on utilise comme support (13) une grille par exemple en matière plastique ou en métal.

15. Capteur selon l'une des revendications précédentes,
caractérisé en ce que le piézo-élément (8) est fixé dans une plaque support (3 ; 33) et est connecté à des surfaces de connexion (16) prolongées en dehors du boîtier; par l'intermédiaire de pistes conductrices (15) impriméés sur la plaque support.

16. Capteur selon l'une des revendications 1 à 15, constitué par un capteur de coagulation du sang,
caractérisé en ce qu'il est prévu comme composant de réaction du CaCl2 ou d'autres substances capables d'influencer le déroulement de la coagulation.

17. Capteur selon l'une des revendications précédentes,
caractérisé en ce qu'il est muni d'un dispositif de réglage de la température pour maintenir la température à une valeur constante.

18. Dispositif de mesure comprenant un capteur selon l'une des revendications précédentes, caractérisé en ce qu'il comprend un circuit oscillant (51) et un circuit à microprocesseur (52) utilisé comme circuit d'analyse électronique (50), en ce que le piézo-élément (8) est ajouté dans le circuit oscillant (51) en tant qu'élément déterminant la fréquence, et en ce que les variations de fréquence sont mesurées et analysées en numérique par le circuit à microprocesseur (52).

19. Dispositif de mesure selon la revendication 18, caractérisé en ce que la sensibilité du piézo-élément (8) à la température est mise à profit pour mesurer la température.
